# EUROPEAN PATENT SPECIFICATION

(11) **EP 3 230 711 B1**
(45) Date of publication and mention of the grant of the patent: **25.04.2018**
(21) Application number: 15816830.2
(22) Date of filing: 07.12.2015
(51) Int. Cl.: G01N 3/38, C12M 1/42, H01F 5/00, H01F 7/13

(54) **BIOLOGICAL SAMPLE ACTUATOR**
AKTUATOR FÜR BIOLOGISCHE PROBEN
ACTIONNEUR D'ÉCHANTILLON BIOLOGIQUE

(30) Priority: 11.12.2014 GB 201422016
(43) Date of publication of application: 18.10.2017
(73) Proprietor: Oxford University Innovation Limited, Botley Oxford OX2 0JB (GB)
(72) Inventor: BURTON, Jonathan Joshua, Kington Herefordshire HR5 3HS (GB); MCCULLOCH, Malcolm, Oxford Oxfordshire OX2 0NG (GB); THOMPSON, Mark Stuart, Oxford Oxfordshire OX1 3PN (GB); STEVENS, Chris, Oxford Oxfordshire OX1 3PJ (GB)
(74) Representative: Barker Brettell LLP
(86) International application number: PCT/GB2015/053738
(87) International publication number: WO 2016/092278

(56) References cited:
- US-A- 521 269
- US-A1- 2013 160 577
- ISAIAH ADEKANMBI ET AL: "A novel in vitro loading system for high frequency loading of cultured tendon fascicles", MEDICAL ENGINEERING & PHYSICS., vol. 35, no. 2, 1 February 2013 (2013-02-01), pages 205-210, XP055255894, GB ISSN: 1350-4533, DOI: 10.1016/j.medengphy.2012.08.015 cited in the application
- Nilangshu K. Das ET AL: "DESIGN OF MINIATURE COIL TO GENERATE UNIFORM MAGNETIC FIELD", Progress In Electromagnetics Research M, vol. 34, 20 December 2013 (2013-12-20), pages 99-105, XP055255479, US ISSN: 1937-8726, DOI: 10.2528/PIERM13112602

## Description

The invention relates to an actuator for mechanically loading a biological sample.

Biological tissue may respond to mechanical loading. For example, exercise can significantly enhance bone strength at loaded sites in children¹. Collagen turnover in tendons in humans is related to levels of activity: inactivity tending to decrease collagen turnover².
¹ Nikander, Riku, et al. "Targeted exercise against osteoporosis: A systematic review and meta-analysis for optimising bone strength throughout life." BMC medicine 8.1 (2010): 47
² Kjær, Michael. "Role of extracellular matrix in adaptation of tendon and skeletal muscle to mechanical loading." Physiological reviews 84.2 (2004): 649-698)

In the field of regenerative medicine there is a need to understand the mechanisms by which tissue responds to physical activity. One aspect of this response is how tissue responds to mechanical loading. Understanding the response of tissue in vivo is complicated, because the environment is very complex and difficult to control. The ability to subject tissue to mechanical loading in vitro potentially enables more control over the environment.

One application for in vitro mechanical loading of biological samples is to perform basic science, allowing specific hypotheses to be tested in a controlled manner. Mechanical loading of tissues may provide a more representative arrangement for drug testing on such tissues, contributing to reducing and replacing animal testing in medicine. Furthermore, arrangements for mechanical loading of tissue may facilitate improved tissue engineering.

Preferably, an arrangement for mechanical loading would be compatible with maintaining a sterile environment around the biological sample under test, and allow a controlled temperature to be maintained. Furthermore, it is preferable that relatively high cycles of reversed stress can be applied to the sample. Relatively high frequency tissue loading is an active area of research, with whole body vibration at 30Hz having shown promise in increasing tendon stiffness and bone mass^{3,4}.
³ Rubin C, Turner AS, Bain S, Mallinckrot C, McLoed K. Low mechanical signals strengthen long bones. Nature 2001;412:603-4.
⁴ Sandhu E, Mile JD, Dauhners LE, Keller BV, Weinhold PS. Whole body vibration increases area and stiffness of the flexor carpi ulnaris tendon in the rat. J Biomech 2011;44:1189-91

An arrangement for mechanical loading has been reported, in which a tendon is mechanically loaded using a magnet and solenoid⁵. A magnet was secured to the tendon, and then placed near a solenoid. The force applied to the tendon was varied by varying the current through the solenoid. A load cell was used to determine the applied load.
⁵ Adekanmbi, Isaiah, Sarah Franklin, and Mark S. Thompson. "A novel in vitro loading system for high frequency loading of cultured tendon fascicles. "Medical engineering & physics 35.2 (2013): 205-210.

WO2013095834 discloses a system for applying mechanical stimulation to a biological sample. The biological sample is clamped at each end to a holder, and a force is directly exerted on the sample via the holder.

A simplified arrangement would be preferable, as would an arrangement with lower cost. One problem with prior art arrangements for mechanical tissue loading is consistency of loading.

An arrangement for mechanical tissue loading that addresses at least some of the above mentioned problems is desired.

According to the invention, there is provided an apparatus for mechanically loading a biological sample, comprising: a hermetically sealable container, for housing the biological sample; a ferromagnetic element, for attachment to the biological sample within the container; and a solenoid for generating a magnetic field within the container, so as to apply a force to the ferromagnetic element, the solenoid having a solenoid axis; wherein the solenoid is configured, when energised by a constant current, to produce a force on the ferromagnetic element that varies by less than a predetermined amount over a predetermined range of movement of the ferromagnetic element within the container; and the solenoid has a hole or recess along the axis for at least partially receiving the container and wherein the container is positioned at least partly within the solenoid so that the ferromagnetic element within the container is within the solenoid. The predetermined amount may be 50%, 30%, 20%, 10%, 5% or 2%.

The predetermined range of movement may be at least 50mm, 40mm, 30mm, 20mm, 10mm or 5mm.

The improved uniformity of force over distance results in improved consistency of mechanical stimulation of a biological sample. The impact of any errors in the initial position of the ferromagnetic element within the solenoid are minimised. Similarly, the effect of any changes in dimension of the sample on the mechanical loading of the sample.

The solenoid may be configured to produce a field gradient that varies by less than a further predetermined amount over a further predetermined distance along the solenoid axis.

The further predetermined amount may be 30%, 20%, 10%, 5% or 2%.

The further predetermined distance may be at least 50mm, 40mm, 30mm, 20mm, 10mm or 5mm.

Where the ferromagnetic element is small (relative to the range of movement), one way of approximating a force that is constant over a range of movement is to configure the solenoid to produce a field gradient that is relatively uniform over the desired range of movement.

The solenoid can be configured with the necessary field distribution to achieve the desired uniformity of force (for a given current) over a desired range of movement of the ferromagnetic element in a number of ways.

The solenoid may comprise a winding having a radius that varies with position along the solenoid axis. Varying the radius of the winding may be achieved by varying the radius of a former or bobbin around which the winding of the solenoid is wound.

The radius of the winding may increase with distance from the centre of the solenoid. This is a convenient way of providing for a more uniform field gradient.

The solenoid may comprise a winding that has a number of winding layers that varies with position along the solenoid axis. For instance, the number of winding layers may be greater in the centre of the solenoid, so as to produce a more uniform field gradient within the solenoid.

The solenoid may comprise a winding has a pitch that varies with position along the solenoid axis. For example, the pitch of the winding may decrease away from the centre of the solenoid, so as to produce a more uniform field gradient within the solenoid.

The apparatus may comprise means for cooling the solenoid by forced convection of a cooling fluid. Actively cooling the solenoid enables it to handle greater currents without overheating, increasing the amount of force that can be applied on the ferromagnetic element.

The fluid used for cooling may be air or water, or any other suitable fluid.

The container may be a sterile container.

The container may comprise a removable lid.

The lid may comprise attachment means, for attachment to the biological sample. The attachment means may comprise a post, clamp, grip or the like.

The ferromagnetic element may comprise a permanent magnet. The use of a magnet may enhance the amount of force that can be applied on the biological sample by the solenoid. A high field strength permanent magnet is particularly suitable for increasing the force.

The ferromagnetic element may comprise a biologically inert coating. The use of a biologically inert coating may facilitate mechanical loading of tissue, without substantially affecting the viability of the tissue.

The coating may be a polymeric material, for instance a flourinated polymer such as polytetraflouroethylene (PTFE).

The diameter of the hole of the solenoid may be at least 10mm. The diameter of the hole may be at least 20mm, 30mm, 40mm, or 50mm.

The depth of the hole may be at least 20mm. The hole may be a through hole. The depth of the hole may be at least 30mm, 40mm, 50mm. 75mm or 100mm.

The length of the solenoid may be at least 50mm, 60mm, 75mm or 100mm.

According to second aspect of the invention, there is provided a method of mechanically loading a biological sample, comprising: attaching a first portion of a the biological sample to a fixed object; attaching a ferromagnetic element to a second portion of the biological sample; housing the biological sample and ferromagnetic element with a container; positioning the ferromagnetic element within a working region of a solenoid; and exerting a force on the ferromagnetic element using a solenoid; wherein the solenoid is configured, when energised by a constant current, to produce a force on the ferromagnetic element that varies by less than a predetermined amount over a predetermined range of movement of the ferromagnetic element within the container; and wherein
the solenoid has a solenoid axis and a hole or recess along the axis for at least partially receiving the container so that the ferromagnetic element within the container is within the solenoid and wherein
positioning the ferromagnetic element within the working region of the solenoid comprises placing the container at least partially within the hole or recess. Any of the features of the first aspect may be included in the second aspect of the invention.

The method may comprise using the apparatus according to any preceding claim.

The method may include a step of testing a substance for use a medicament on the biological sample while it is loaded periodically using the solenoid.

The biological sample may be tissue, and the method may comprise conditioning the tissue using the force exerted by the solenoid.

Example embodiments of the invention will now be described, with reference to the accompanying drawings, in which:
Figure 1 is a schematic diagram of an apparatus according to an embodiment of the invention;
Figure 2 is a graph of the magnetic field and field gradient along the axis of a conventional solenoid;
Figures 3, 4 and 5 show windings with varying pitch, varying number of layers, and varying radius, respectively;
Figure 6 is a schematic diagram of an apparatus according to an embodiment of the invention;
Figure 7 is a graph of solenoid winding radius with respect to the distance from the solenoid centre along the solenoid axis, according to an embodiment,
Figure 8 is a schematic diagram of a solenoid former in accordance with Figure 7;
Figure 9 is graph of the modelled magnetic field with respect to the distance from the solenoid centre along the solenoid axis for the embodiment of Figures 7 and 8;
Figure 10 is a graph of the variation from a uniform field gradient for a range of distances along the solenoid axis, from 1cm to 4cm from the solenoid centre; and
Figure 11 is a schematic diagram of an alternative solenoid design, produced by optimisation via finite element modelling; and
Figure 12 is a graph of the field gradient for the solenoid of Figure 11.

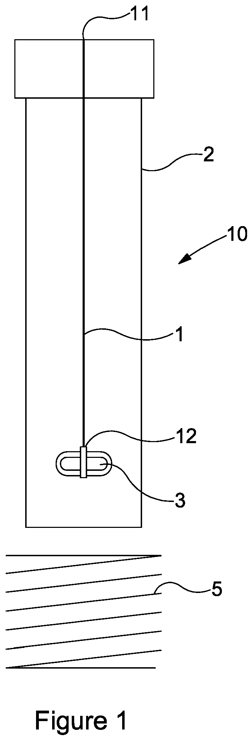

Referring to Figure 1, an arrangement 10 for mechanically loading a biological sample 1 is shown. The biological sample 1, in this case a tendon, is attached to the interior of a container 2 at a first location 11. At a second location 12, the sample 1 is attached to a magnet 3. The container 2 is then positioned over a solenoid 5 and a current passed through the solenoid 5 to control the force exerted by the magnet 3 on the sample 1.

In prior art arrangements a conventional solenoid 5 is used. The applicant has identified that a conventional solenoid 5 is highly sensitive to the position of the sample. It can be difficult to accurately position the sample within the container 2, because some of the sample 1 may be taken up by attaching it to the container 2 and magnet 3. Furthermore, the length of the sample 1 may change, for example as a result of creep from mechanical loading.

It is possible to compensate, at least to some extent, for the positional sensitivity of the actuation force by using closed loop control. Monitoring the force applied to the sample using load cell allows a current to be applied to the solenoid that produces a required force at the magnet. However, this approach requires a relatively expensive control loop and force transducer. Furthermore, it can only compensate for the positional sensitivity to a point - in some relative positions of magnet and solenoid, it may no longer be possible to exert the required force.

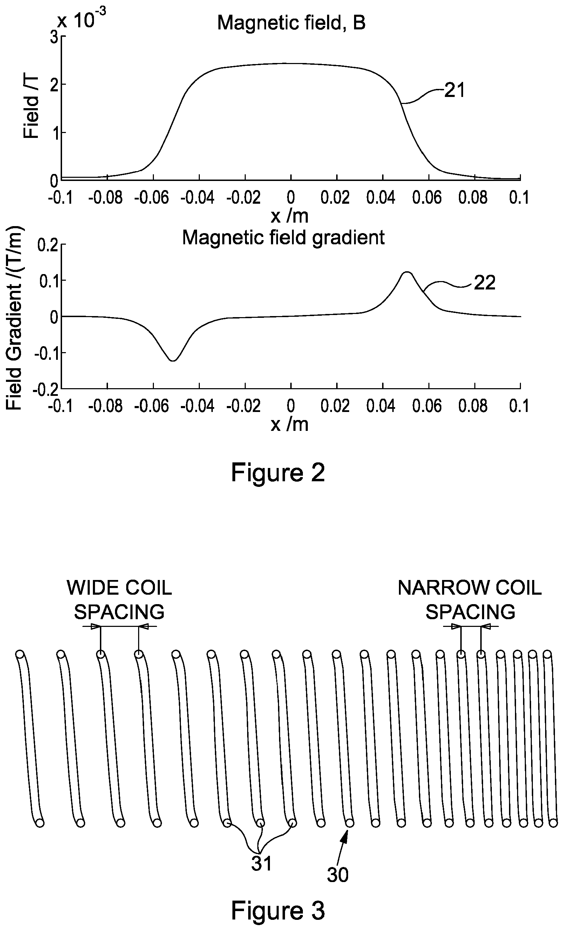

Figure 2 shows a graph of modelled magnetic field 21 and magnetic field gradient 22 for an example conventional solenoid. The length of the solenoid is 100mm, the winding radius is 10mm, the winding has 51 turns, and the modelled current is 1 amp. It can be seen that the rate of change of magnetic field gradient with position is significant, and this is particularly pronounced where the field gradient is high (near the ends of the solenoid, at x~±50mm). This can be problematic in the case of mechanically loading a biological sample, because it results in inconsistency of loading. The present applicant has identified that it can be difficult to precisely position a ferromagnetic element (such as the magnet 3) when it is supported by a biological sample. It can be difficult to secure a biological sample 1 to the container 2 and magnet 3 such that the location of the magnet 3 within the container 2 is sufficiently accurate.

Furthermore, as the biological sample 1 undergoes mechanical loading, it may deform to a significant effect, for example as a result of creep. Even if the initial position of the magnet 3 is accurately defined within the container 2, as mechanical loading progresses over time, the location of the magnet 3 may move to a location in which the field gradient is substantially different than the initial position. The applicant has identified that it is this characteristic of the solenoid that results in the problematic inconsistency of loading of the biological sample.

According to an embodiment of the invention, the problem of inconsistency of mechanically loading a biological sample is solved by the use of a solenoid and ferromagnetic element in which the solenoid is configured to provide a more consistent force characteristic over a suitable distance.

This more consistent force characteristic can be approximated by configuring the solenoid to have a substantially uniform field gradient over a sufficient distance. For example, a solenoid with a field gradient that varies by less than 10% over a distance of at least 20mm may provide a relatively uniform force (for a given current) on a ferroelectric element over that range of movement within the magnetic field of the solenoid. This configuration may be particularly applicable where the ferromagnetic element is relatively small.

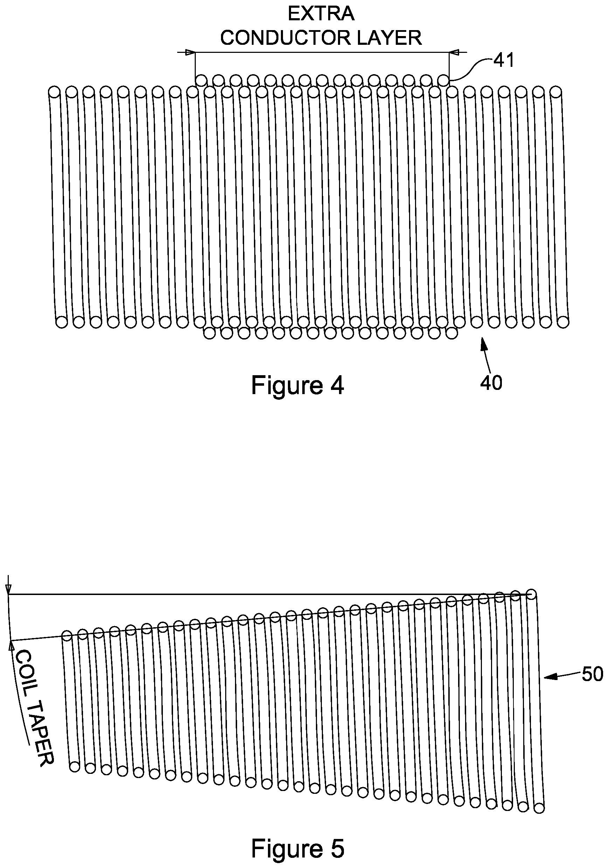

Figures 3 to 5 show examples of design parameters of a solenoid that affect the field of the solenoid. Increasing the pitch between adjacent turns 31 of a solenoid winding 30, as shown in Figure 3, will decrease the magnetic field with increasing pitch. Adding additional layers of turns 41, as shown in Figure 4, will increase the magnetic field in proportion to the number of layers. Adjusting the radius of the turns (or tapering the coil), as shown in Figure 5, will increase the strength of the magnetic field at the solenoid axis with decreasing radius of the turns.

The relationship between such design parameters and the magnetic field and field gradient may be determined by methods such as finite element analysis and analytical approximations based on the Biot-Savart law.

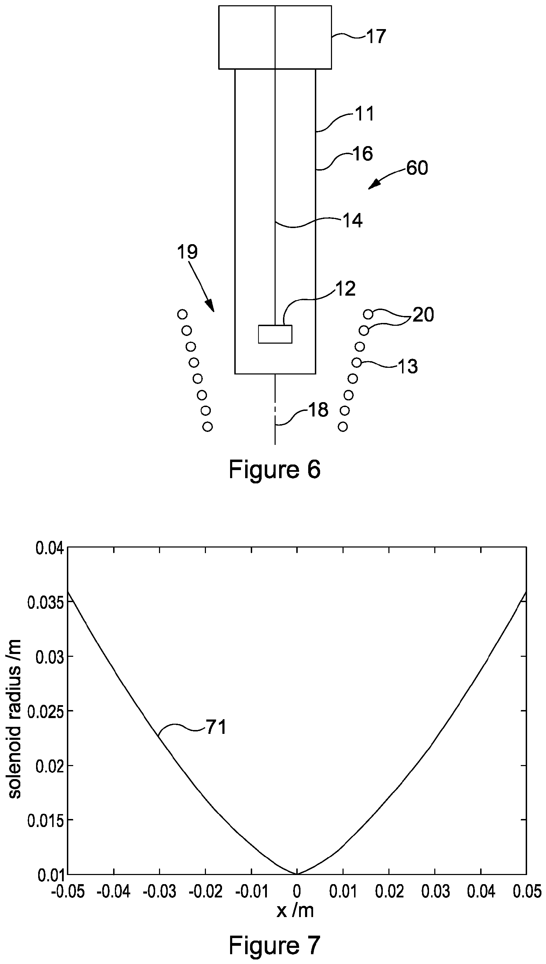

Referring to Figure 6, an apparatus 60 according to an embodiment is schematically illustrated, comprising a container 11, ferromagnetic element 12, and solenoid 13. A biological sample 14 is attached between the ferromagnetic element 12 and the container 11. In this example the biological sample is attached to the lid 17 of the container, but this is not essential, and the biological sample 14 may be supported in other configurations. For instance, the biological sample 14 could be supported by the container 11 at a first and second location, and the ferromagnetic element 12 supported by a portion of the biological sample 13 that is between the first and second location (e.g. in a lateral bridge configuration).

The container 11 comprises a body 16 and lid 17. The lid 17 is sealably engagable with the body 16, so that a sterile environment may be formed housing the biological sample 14. The container 11 may be substantially cylindrical. The container 11 may comprise a test tube or centrifuge tube with a screw cap. The container 11 may be sterile.

The biological sample 14 may comprise tissue. The tissue may be cultured in vitro, or obtained by a biopsy or similar procedure from an organism. The biological sample 14 may comprise any biological sample. In one example the biological sample may be fascicles, such as tendon fascicles. The biological sample 14 may be surrounded by a growth or culture medium, for keeping the biological sample 14 viable.

The solenoid 13 in this example is configured to produce a field gradient that is more linear by varying the radius of the turns 20 of the winding with the location along the solenoid axis 18. The radius of the turns 20 increases towards the end of the solenoid 19 that is facing the container 11, so that the magnetic field on the axis of the solenoid 13 decreases towards the turns 20 with a larger radius.

The container 11 may be positioned completely outside the solenoid 13 (not shown), or positioned partly within the solenoid 13, as shown in Figure 6. The solenoid may include a recess or through hole to facilitate this.

The ferromagnetic element 12 may comprise a permanent magnet, such as a rare earth magnet (e.g. cobalt samarium or neodymium). The ferromagnetic element 12 may be encapsulated with a biologically inert coating, which may be polymeric. An example of such a coating is polytetraflouroethylene (PTFE).

In this embodiment the field gradient of the solenoid 13 on the solenoid axis 18 is adjusted by varying the radius of the turns 20 of the solenoid. In other embodiments the pitch of the turns 20, or the number of layers in the solenoid 13 may be varied along the length of the solenoid 13 to achieve reduced variation in force as a function of the position of the ferromagnetic element 12. The turn pitch, number of layers and turn radii can each be adjusted independently to achieve the required coil configuration, or may be varied in any combination.

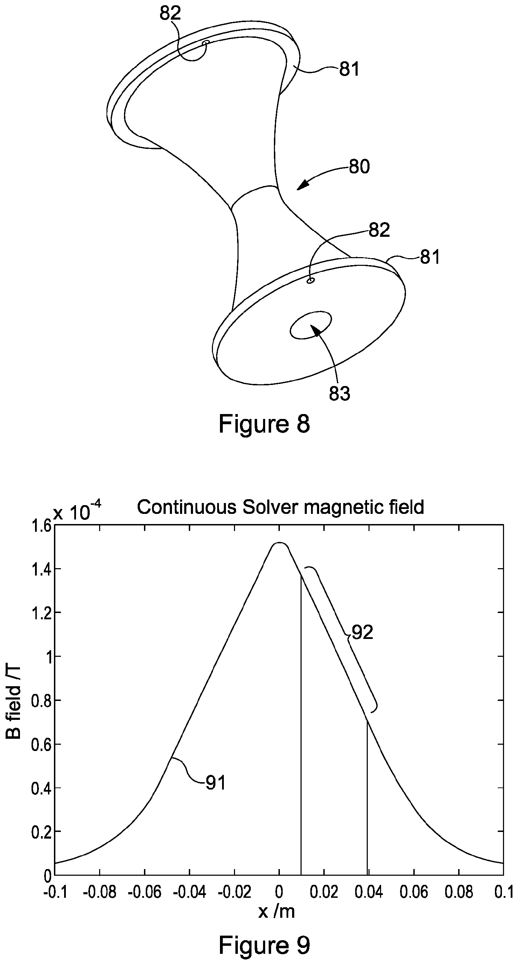

An illustrative example of a solenoid 13 that is suitable for providing a substantially uniform force on a ferromagnetic element over a particular distance (for a particular current flowing through the solenoid) is shown in Figures 7 and 8.

In this embodiment a uniform pitch of the turns is assumed, along with a uniform number of layers. The radius of the solenoid winding is varied so as to achieve a field gradient that varies by less than a predetermined amount along the axis of the solenoid over a predetermined distance. In this embodiment, the design of the solenoid was optimised to approximate a substantially uniform field gradient over a distance of between 10mm and 40mm from the centre of the solenoid.

Referring to Figure 7, a curve 71 of the radius of the solenoid winding is shown with respect to the distance along the solenoid axis. In this example a minimum solenoid radius of 10mm was selected, to allow the container to be positioned at least partly within the solenoid.

Figure 8 shows a solid model of a solenoid former 80 in accordance with the graph of Figure 7. Shoulders 81 are provided at the ends of the former 30, and through holes 82 are provided through these for securing the start and end of the solenoid winding. A through hole 83 is provided along the axis of the solenoid, for at least partially receiving the container.

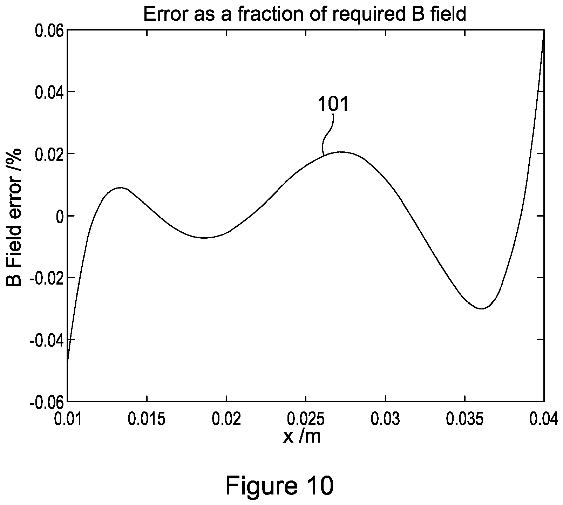

Figure 9 shows a curve 91 of calculated magnetic field on the solenoid axis for the solenoid design shown in Figures 7 and 8. It is clear that the graph is very linear in the optimised region 92 between a distance of 10mm and 40mm from the centre of the solenoid. Figure 10 shows a curve 101 of the difference between the magnetic field of the solenoid of Figures 7 and 8 and the linear target function against which the design was designed. An error of 0% would represent a (calculated) fixed field gradient for 10mm<x<40mm.

The design shown in Figures 7 and 8 is calculated to achieve a field gradient that varies by less than 0. 1% over a predetermined distance of 30mm (from x=10mm to x=40mm). It will be understood that a real solenoid made to this design may not produce a field that perfectly matches that calculated in Figures 9 and 10, but it can be expected that a relatively uniform force on a ferromagnetic element will result from the uniform field gradient over the predetermined distance. The ferromagnetic element may be arranged to extend over less than a fifth of the predetermined distance when the ferromagnetic element is supported by the biological sample. For instance, the ferromagnetic element for the embodiment of Figures 7 and 8 may have a length of 6mm or less along the solenoid axis, when supported by a tissue sample.

Although a specific embodiment has been described in which a solenoid radius was varied to approximate a linear field gradient over a predetermined distance within the solenoid, in other examples the solenoid may be optimised with a particular ferromagnetic element in mind, so as to produce a particularly uniform force response with respect to the location of the ferromagnetic element along the solenoid axis.

Although a relatively small ferromagnetic element and a uniform field gradient is one way to achieve a more constant force characteristic over distance, an alternative approach is possible in which a ferromagnetic element extends over a greater distance along the solenoid axis. The appropriate configuration of the solenoid to approximate a constant force over a predetermined range of movement of the ferromagnetic element may thereby take into account the interaction of the ferromagnetic element with the magnetic field of the solenoid. Where the ferromagnetic element is a permanent magnet, the interaction between the distributed field of the magnet and that of the solenoid may be taken into account in optimising the solenoid to better approximate uniform force over a range of displacement.

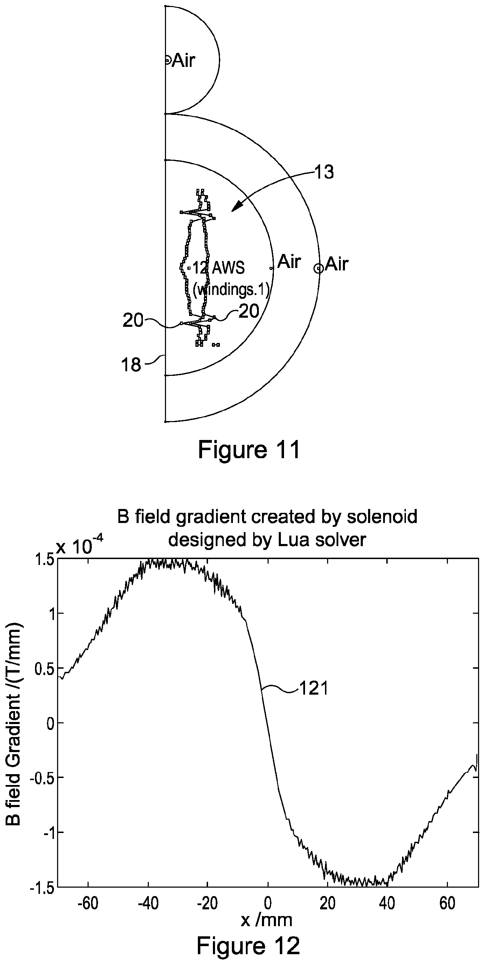

Referring to Figure 11, an alternative configuration for solenoid 13 is shown, in which the position of each turn of the windings 20 is illustrated with respect to the solenoid axis 18. This configuration was produced by iterative finite element modelling, using Lua scripting of FEMM (Finite Element Method Magnetics).

Figure 12 shows a curve 121 of the modelled field gradient for the solenoid 13 of Figure 11. The modelled field gradient is relatively uniform between x=20mm and x=40mm, varying by less than 15% over this range.

The maximum force that can be produced on the ferromagnetic element by the solenoid is limited by the current that can flow through the solenoid without overheating it. This current limit can be increased by improving the cooling of the solenoid. This can be achieved in a number of ways, for instance: by using a material with high thermal conductivity for the bobbin or former; by providing structures to improve heat transfer to the ambient environment (e.g. fins and ridges); or by forced convection of a cooling fluid. The cooling fluid may be air or a liquid. which may comprise water. A fan or impeller may be provided to draw the fluid over the solenoid, thereby improving heat transfer from the solenoid winding. This may improve the maximum force that can be applied on the magnet using the solenoid.

The preceding examples are not intended to limit the scope of the invention. A number of variations are possible, within the scope of the invention, as determined by the appended claims.

## Claims

1. An apparatus for mechanically loading a biological sample, comprising:
a hermetically sealable container, for housing the biological sample;
a ferromagnetic element, for attachment to the biological sample within the container; and
a solenoid for generating a magnetic field within the container, so as to apply a force to the ferromagnetic element, the solenoid having a solenoid axis;
wherein: the solenoid is configured, when energised by a constant current, to produce a force on the ferromagnetic element that varies by less than a predetermined amount over a predetermined range of movement of the ferromagnetic element within the container;
**characterized in that** the solenoid has a hole or recess along the axis for at least partially receiving the container and **in that** the container is positioned at least partly within the solenoid so that the ferromagnetic element within the container is within the solenoid.

2. The apparatus of claim 1, wherein the solenoid is configured to produce a field gradient that varies by less than 50% over a distance of at least 10mm along the solenoid axis, or less than 20% over a distance of at least 10mm along the solenoid axis.

3. The apparatus of claim 1 or 2, wherein the solenoid comprises a winding with a radius that varies with position along the solenoid axis.

4. The apparatus of claim 3, wherein the radius of the winding increases with distance from the centre of the solenoid.

5. The apparatus of any preceding claim, wherein the solenoid comprises a winding that has a number of winding layers that varies with position along the solenoid axis.

6. The apparatus of any preceding claim, wherein the solenoid comprises a winding has a pitch that varies with position along the solenoid axis.

7. The apparatus of any preceding claim, further comprising means for cooling the solenoid by forced convection of a cooling fluid.

8. The apparatus of any preceding claim, wherein the container is a sterile container.

9. The apparatus of any preceding claim, wherein the container comprises a removable lid, and the lid comprises attachment means, for attachment to the biological sample.

10. The apparatus of any preceding claim, wherein the ferromagnetic element comprises a permanent magnet and/or a biologically inert coating.

11. The apparatus of any preceding claim, wherein the diameter of the hole or recess is at least 10mm, or at least 20mm, or at least 50mm.

12. A method of mechanically loading a biological sample, comprising:
attaching a first portion of the biological sample to a fixed object;
attaching a ferromagnetic element to a second portion of the biological sample;
housing the biological sample and ferromagnetic element with a container;
positioning the ferromagnetic element within a working region of a solenoid; and
exerting a force on the ferromagnetic element using a solenoid;
wherein:
the solenoid is configured, when energised by a constant current, to produce a force on the ferromagnetic element that varies by less than a predetermined amount over a predetermined range of movement of the ferromagnetic element within the container; and **characterized in that**
the solenoid has a solenoid axis and a hole or recess along the axis for at least partially receiving the container so that the ferromagnetic element within the container is within the solenoid and **in that** positioning the ferromagnetic element within the working region of the solenoid comprises placing the container at least partially within the hole or recess.

13. The method of claim 12, wherein the method comprises using the apparatus according to any of claims 1 to 11.

14. The method of any claim 12 or 13, including a step of testing a substance for use a medicament on the biological sample while it is loaded periodically using the solenoid.

15. The method of any of claims 12 to 14, wherein the biological sample is tissue, and the method comprises conditioning the tissue using the force exerted by the solenoid.

## Patentansprüche

1. Vorrichtung zum mechanischen Laden einer biologischen Probe, umfassend:
einen hermetisch verschließbaren Behälter zum Unterbringen der biologischen Probe;
ein ferromagnetisches Element zum Befestigen an der biologischen Probe innerhalb des Behälters; und
ein Solenoid zum Erzeugen eines magnetischen Felds innerhalb des Behälters, um eine Kraft auf das ferromagnetische Element aufzubringen, wobei das Solenoid eine Solenoidachse aufweist;
wobei: das Solenoid konfiguriert wird, wenn es durch einen konstanten Strom erregt wird, um eine Kraft auf dem ferromagnetischen Element zu erzeugen, die weniger als eine vorbestimmte Menge über einen vorbestimmten Bewegungsbereich des ferromagnetischen Elements innerhalb des Behälters variiert;
**dadurch gekennzeichnet, dass** das Solenoid ein Loch oder eine Vertiefung der Achse entlang zum mindestens teilweisen Aufnehmen des Behälters aufweist und dass der Behälter zumindest teilweise innerhalb des Solenoids positioniert ist, so dass das ferromagnetische Element innerhalb des Behälters innerhalb des Solenoids liegt.

2. Vorrichtung nach Anspruch 1, wobei das Solenoid konfiguriert ist, um einen Feldgradienten zu erzeugen, der weniger als 50 % über eine Entfernung von mindestens 10 mm der Solenoidachse entlang oder weniger als 20 % über eine Entfernung von mindestens 10 mm der Solenoidachse entlang variiert.

3. Vorrichtung nach Anspruch 1 oder 2, wobei das Solenoid eine Wicklung mit einem Radius umfasst, der der Position der Solenoidachse entlang entsprechend variiert.

4. Vorrichtung nach Anspruch 3, wobei der Radius der Wicklung mit der Entfernung von der Mitte des Solenoids zunimmt.

5. Vorrichtung nach einem der vorstehenden Ansprüche, wobei das Solenoid eine Wicklung aufweist, die eine Anzahl von Wicklungsschichten aufweist, die mit der Position der Solenoidachse entlang variiert.

6. Vorrichtung nach einem der vorstehenden Ansprüche, wobei das Solenoid eine Wicklung umfasst, die einen Abstand aufweist, der mit der Position der Solenoidachse entlang variiert.

7. Vorrichtung nach einem der vorstehenden Ansprüche, weiter umfassend Mittel zum Kühlen des Solenoids durch Zwangskonvektion eines Kühlfluids.

8. Vorrichtung nach einem der vorstehenden Ansprüche, wobei der Behälter ein steriler Behälter ist.

9. Vorrichtung nach einem der vorstehenden Ansprüche, wobei der Behälter einen entfernbaren Deckel umfasst und der Deckel ein Befestigungsmittel zum Befestigen der biologischen Probe umfasst.

10. Vorrichtung nach einem der vorstehenden Ansprüche, wobei das ferromagnetische Element einen Permanentmagnet und/oder eine biologisch inerte Beschichtung umfasst.

11. Vorrichtung nach einem der vorstehenden Ansprüche, wobei der Durchmesser des Lochs oder der Vertiefung mindestens 10 mm oder mindestens 20 mm oder mindestens 50 mm beträgt.

12. Verfahren zum mechanischen Laden einer biologischen Probe, umfassend:
Befestigen eines ersten Teils der biologischen Probe an einem fixierten Objekt;
Befestigen eines ferromagnetischen Elements an einem zweiten Teil der biologischen Probe;
Unterbringen der biologischen Probe und des ferromagnetischen Elements in einem Behälter;
Positionieren des ferromagnetischen Elements innerhalb eines Arbeitsbereichs eines Solenoids; und
Ausüben einer Kraft auf das ferromagnetische Element unter Anwendung eines Solenoids;
wobei: das Solenoid konfiguriert wird, wenn es durch einen konstanten Strom erregt wird, um eine Kraft auf dem ferromagnetischen Element zu erzeugen, die weniger als eine vorbestimmte Menge über einen vorbestimmten Bewegungsbereich des ferromagnetischen Elements innerhalb des Behälters variiert; und
**dadurch gekennzeichnet, dass** das Solenoid eine Solenoidachse und ein Loch oder eine Vertiefung der Achse entlang zum mindestens teilweisen Aufnehmen des Behälters aufweist, so dass das ferromagnetische Element innerhalb des Behälters innerhalb des Solenoids liegt und dass das Positionieren des ferromagnetischen Elements innerhalb des Arbeitsbereichs des Solenoids das Positionieren des Behälters mindestens teilweise innerhalb des Lochs oder der Vertiefung umfasst.

13. Verfahren nach Anspruch 12, wobei das Verfahren das Verwenden der Vorrichtung nach einem der Ansprüche 1 bis 11 umfasst.

14. Verfahren nach einem der Ansprüche 12 oder 13, umfassend einen Schritt des Testens einer Substanz bezüglich der Verwendung als Medikament an der biologischen Probe, während sie periodisch unter Anwendung des Solenoids geladen wird.

15. Verfahren nach einem der Ansprüche 12 bis 14, wobei die biologische Probe Gewebe ist und das Verfahren das Konditionieren des Gewebes unter Anwendung der Kraft, die von dem Solenoid ausgeübt wird, umfasst.

## Revendications

1. Appareil de chargement mécanique d'un échantillon biologique, comprenant :
un récipient hermétiquement scellable pour recevoir l'échantillon biologique ;
un élément ferromagnétique pour fixer à l'échantillon biologique dans le récipient ; et
un solénoïde pour générer un champ magnétique dans le récipient de manière à appliquer une force à l'élément ferromagnétique, le solénoïde ayant un axe de solénoïde ;
dans lequel le solénoïde est configuré, lorsqu'il est excité par un courant constant, pour produire une force sur l'élément ferromagnétique qui varie de moins d'une quantité prédéterminée sur une plage prédéterminée de mouvement de l'élément ferromagnétique dans le récipient ;
**caractérisé en ce que** le solénoïde a un trou ou une cavité le long de l'axe pour recevoir au moins en partie le récipient et le récipient est positionné au moins en partie dans le solénoïde de sorte que l'élément ferromagnétique dans le récipient se trouve dans le solénoïde.

2. Appareil selon la revendication 1, dans lequel le solénoïde est configuré pour produire un gradient de champ qui varie de moins de 50 % sur une distance d'au moins 10 mm le long de l'axe du solénoïde ou de moins de 20 % sur une distance d'au moins 10 mm le long de l'axe du solénoïde.

3. Appareil selon la revendication 1 ou 2, dans lequel le solénoïde comprend un enroulement avec un rayon qui varie avec la position le long de l'axe du solénoïde.

4. Appareil selon la revendication 3, dans lequel le rayon de l'enroulement augmente avec la distance depuis le centre du solénoïde.

5. Appareil selon l'une quelconque des revendications précédentes, dans lequel le solénoïde comprend un enroulement qui a un nombre de couches d'enroulement qui varie avec la position le long de l'axe du solénoïde.

6. Appareil selon l'une quelconque des revendications précédentes, dans lequel le solénoïde comprend un enroulement qui a un pas qui varie avec la position le long de l'axe du solénoïde.

7. Appareil selon l'une quelconque des revendications précédentes, comprenant en outre des moyens pour refroidir le solénoïde par convexion forcée d'un fluide de refroidissement.

8. Appareil selon l'une quelconque des revendications précédentes, dans lequel le récipient est un récipient stérile.

9. Appareil selon l'une quelconque des revendications précédentes, dans lequel le récipient comprend un couvercle amovible et le couvercle comprend des moyens de fixation pour se fixer à l'échantillon biologique.

10. Appareil selon l'une quelconque des revendications précédentes, dans lequel l'élément ferromagnétique comprend un aimant permanent et/ou un revêtement biologiquement inerte.

11. Appareil selon l'une quelconque des revendications précédentes, dans lequel le diamètre du trou ou de la cavité est d'au moins 10 mm ou d'au moins 20 mm ou d'au moins 50 mm.

12. Procédé de chargement mécanique d'un échantillon biologique, comprenant :
la fixation d'une première partie de l'échantillon biologique à un objet fixe ;
la fixation d'un élément ferromagnétique à une seconde partie de l'échantillon biologique ;
le logement de l'échantillon biologique et de l'élément ferromagnétique avec un récipient ;
le positionnement de l'élément ferromagnétique dans une région de travail d'un solénoïde ; et
l'exercice d'une force sur l'élément ferromagnétique en utilisant un solénoïde ;
dans lequel :
le solénoïde est configuré, lorsqu'il est excité par un courant constant, pour produire une force sur l'élément ferromagnétique qui varie de moins d'une quantité prédéterminée sur une plage prédéterminée de mouvement de l'élément ferromagnétique dans le récipient ; et
**caractérisé en ce que** le solénoïde a un axe de solénoïde et un trou ou une cavité le long de l'axe pour recevoir au moins en partie le récipient de sorte que l'élément ferromagnétique dans le récipient se trouve dans le solénoïde et le positionnement de l'élément ferromagnétique dans la région de travail du solénoïde comprend la mise en place du récipient au moins en partie dans le trou ou la cavité.

13. Procédé selon la revendication 12, dans lequel le procédé comprend l'utilisation de l'appareil selon l'une quelconque des revendications 1 à 11.

14. Procédé selon l'une quelconque des revendications 12 ou 13, comprenant une étape d'essai d'une substance pour utilisation d'un médicament sur l'échantillon biologique tandis qu'il est chargé périodiquement en utilisant le solénoïde.

15. Procédé selon l'une quelconque des revendications 12 à 14, dans lequel l'échantillon biologique est un tissu et le procédé comprend le conditionnement du tissu en utilisant la force exercée par le solénoïde.
